# FASCICULE DE BREVET EUROPEEN

(11) **EP 1 156 829 B1**
(45) Date de publication et mention de la délivrance du brevet: **09.05.2007**
(21) Numéro de dépôt: 00906438.7
(22) Date de dépôt: 21.02.2000
(51) Int. Cl.: A61K 47/14, A61K 9/48, A61K 9/08, A61K 31/336, A61P 33/02

(54) **FORMULATION STABLE CONTENANT DE LA FUMAGILLINE**
STABILE FUMAGILLIN-FORMULIERUNG
STABLE FORMULATION CONTAINING FUMAGILLIN

(30) Priorité: 26.02.1999 FR 9902401; 27.07.1999 FR 9909702
(43) Date de publication de la demande: 28.11.2001
(73) Titulaire: Sanofi-Aventis, 75013 Paris (FR)
(72) Inventeur: ABRAMOVICI, Bernard, 34990 Juvignac (FR); DUBOIS, Jean-Luc, 34270 Vacquières (FR)
(74) Mandataire: Monain, Patrice
(86) Numéro de dépôt international: PCT/FR2000/000424
(87) Numéro de publication internationale: WO 2000/050084

(56) Documents cités:
- EP-A- 0 799 616
- WO-A-96/30010
- US-A- 4 918 103

## Description

La présente invention concerne une formulation stable de la fumagilline. En particulier la présente invention concerne une formulation stable de la fumagilline pour administration orale ou rectale.

La fumagilline est un antibiotique décrit pour la première fois en 1951 (The Merck Index, 12^{th} Edition, n. 4308). Initialement, la fumagilline était utilisée pour prévenir ou contrôler les maladies parasitaires dans les élevages de poissons et d'abeilles. Plus récemment, la fumagilline s'est révélée d'intérêt en thérapie humaine. En particulier, on pourra se référer à la demande de brevet WO 96/30010 qui souligne l'efficacité de la fumagilline dans le traitement des infections intestinales dues à des microsporidies et/ou cryptosporidies notamment chez les patients atteints par le virus HIV.

La fumagilline, acide 2,4,6,8-décatetraènedioique du mono [4-(1,2-époxy-1,5-diméthyl-4-hexényl)-5-méthoxy-1-oxaspiro[2.5]oct-6-yl] ester de formule: 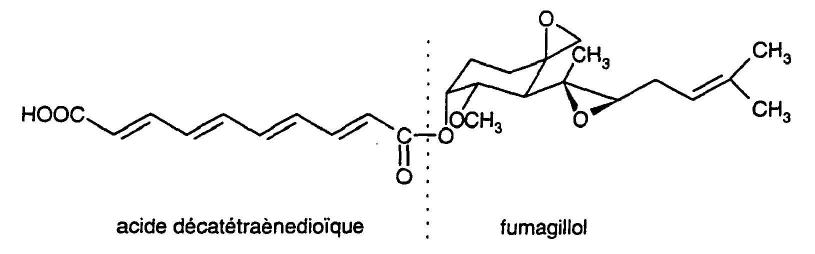 est très instable à température ambiante et doit être conservée à -80°C. La fumagilline présente diverses fonctions instables : instables à température ambiante, instables à la lumière, facilement oxydables ou hydrolysables. On pourra se référer à différentes études effectuées sur la fumagilline, notamment dans : J. Agric. Food Chem. 1991, *39*, 2206-2213 ; J. Pharm. Sci. 1976, *65*(1), 1-22 ; J. Amer. Chem. Soc. 1955, *77*, 5610-5617 et J. Amer. Pharm. Ass., Sci. Ed. 1954, *43*, 536-543. Les différentes dégradations que peut subir la fumagilline sont donc principalement dues à la présence des fonctions époxy, de la fonction ester et des doubles liaisons conjuguées.

Pour que la fumagilline puisse être utilisée comme médicaments, il faut préparer une formulation pharmaceutique stable à température ambiante, facilement stockable.

Certaines compositions stables de dérivés du fumagillol ont déjà été décrites.

Par exemple le brevet EP 602586 décrit des compositions pharmaceutiques stables contenant un dérivé du fumagillol et un ester d'acide gras et de glycérol ou de polyglycérol. La demande de brevet EP 799616 décrit des compositions pharmaceutiques pour administration par voie orale comprenant un dérivé du fumagillol et une base oléagineuse, les dites compositions étant stables contre l'acide gastrique. Aucun de ces deux documents ne décrit spécifiquement une formulation de la fumagilline.

Selon la présente invention, il a maintenant été trouvé que dans le cas de la fumagilline, en utilisant un agent hydrophobe : l'ester de propylèneglycol dicaprate/dicaprylate, on obtient une formulation stable. On obtient ainsi une formulation de la fumagilline stable à température ambiante, sans ajout d'agent stabilisant ou anti-oxydant.

L'ester du propylèneglycol dicaprate/dicaprylate encore nommé acide dicaprylocaprate de propylène glycol est commercialisé sous la marque Labrafac® PG.

Ainsi la présente invention concerne une formulation stable, administrable par voie orale contenant la fumagilline et du propylèneglycol dicaprate/dicaprylate.

En particulier, la formulation selon la présente invention se présente sous la forme d'une gélule ou d'une capsule molle contenant la fumagilline et du propylèneglycol dicaprate/dicaprylate. La gélule ou la capsule est de préférence non-translucide, par exemple colorée ou blanc opaque. Pour assurer l'étanchéité de ces formulations, les dites gélules ou capsules pourront être banderolées ou scellées.

Le procédé LEMS (Liquid Encapsulation by Micro Spray) s'effectue en continu en sortie directe des machines de remplissage des gélules (LEMS capsugel). Il consiste à sceller des gélules Licaps par un procédé de pulvérisation-spray de liquide hydro-alcoolique et de séchage permettant le collage de la gélatine sur elle même entre la corps et la coiffe de la gélule, assurant ainsi l'étanchéité souhaitée.

Dans le procédé classique de banderolage, les gélules provenant des machines de remplissage sont positionnées sur une machine à banderoler comprenant un tapis support de gélules (positionnées à plat dans des alvéoles et en contact avec un disque inférieur). Sous le tapis circulant, sont positionnés des disques verticaux, qui par rotation dans une solution de gélatine viennent déposer entre le corps et la coiffe de la gélule une bande de gélatine ; on peut le cas échéant effectuer un double passage des gélules, c'est à dire un double banderolage car lors du premier passage il peut y avoir génération de micro bulles d'air au niveau de la bande de gélatine déposée ; c'est une opération de sécurité vis à vis de l'étanchéité.

On pourra également utiliser une formulation sous forme d'une gélule (ou capsule) contenant une gélule (ou capsule respectivement) de taille plus petite contenant la fumagilline et le propylèneglycol dicaprate/dicaprylate.

Selon un autre de ses aspects, la présente invention concerne des formulations liquides buvables contenant un mélange de la fumagilline et du propylèneglycol dicaprate/dicaprylate. Ces formulations peuvent se présenter sous la forme de sirop de préférence conservé en flacon multidose ou sous la forme d'un liquide conditionné en ampoule unidose. De préférence, les flacons ou ampoules utilisés sont non-translucides par exemple en verre brun.

La présente invention a également pour objet des formulations stables, administrables par voie rectale, contenant la fumagilline et le propylèneglycol dicaprate/dicaprylate. Ces compositions sont de préférence conditionnées en récipient unidose avec canule.

Les formulations selon l'invention comprennent avantageusement 0,2 à 15% (p/p) de préférence 5 à 12 %(p/p) de fumagilline.

Les formulations selon l'invention peuvent également contenir d'autres excipients pharmaceutiques. On pourra en particulier utiliser un agent thixotrope ou viscosant comme par exemple de la silice colloïdale : Aérosil® R972 ou Aérosil® R974, ou bien l'éthylcellulose, l'Aérosil® R972 étant préféré.

La préparation des formulations est réalisée comme suit : la fumagilline est additionnée au propylèneglycol dicaprate/dicaprylate sous faible agitation afin d'homogénéiser le mélange. Les gélules sont ensuite remplies avec ce mélange au moyen d'une machine semi-automatique.

La présente invention a également pour objet l'utilisation de ces formulations stables de la fumagilline pour la préparation de médicaments destinés à traiter les infections intestinales dues à des microsporidies et/ou à des cryptosporidies ainsi que pour le traitement de ces dites infections.

Plus particulièrement, l'invention concerne l'utilisation de ces formulations chez les malades du SIDA et pour des infections intestinales dont le responsable principal est le parasite *Enterocytozoon bieneusi.*

La dose journalière de principe actif à administrer varie naturellement selon l'âge et le poids du patient, ainsi que selon le type et la gravité de la pathologie à traiter.

En général pour un adulte ayant une constitution normale, la dose journalière de principe actif, à administrer selon l'invention, est comprise entre 1 et 100 mg, de préférence entre 40 et 80 mg, en particulier 60 mg.

Les formes unitaires peuvent contenir de 1 à 100 mg de principe actif avantageusement de 5 à 50 mg ou de préférence de 7 à 30 mg, en particulier 20 mg. De telles doses unitaires sont en général administrées 1 à 4 fois par jour, de préférence 3 fois par jour. Ce type de traitement dure de 1 à 4 semaines, de préférence 2 semaines.

### EXEMPLE 1 :

| Constituant | Formule unitaire |
|---|---|
| Fumagilline | 20 mg |
| Labrafac®PG | 215 mg |

pour une gélule Licaps "blanc-opaque" taille 0 remplie à 235 mg

### Mélange

Le Labrafac®PG est incorporé dans un récipient approprié. Sous très faible agitation, on additionne progressivement le principe actif et agite jusqu'à obtenir une solution homogène.

### Remplissage

Les gélules de type Licaps "blanc-opaque" taille n°0, au poids théorique de 235 mg sont remplies avec le mélange ci-dessus à l'aide d'une machine semi-automatique "HIBAR".

### EXEMPLE 2 :

| Constituant | Formule unitaire |
|---|---|
| Fumagilline | 20 mg |
| Labrafac®PG | 400 mg |

pour une gélule Licaps orange suédois op.C314 taille 0 remplie à 420 mg

### EXEMPLE 3:

| Constituant | Formule unitaire |
|---|---|
| Fumagilline | 20 mg |
| Labrafac®PG | 215 mg |

pour une gélule Licaps orange suédois op.C314 taille 3 remplie à 235 mg

### EXEMPLE 4:

| Constituant | Formule unitaire |
|---|---|
| Fumagilline | 20 mg |
| Aerosil® R972 | 11,75 mg |
| Labrafac®PG | 223,25 mg |

pour une gélule Licaps "blanc-opaque" taille 3 remplie à 250 mg.

### EXEMPLE 5 :

| Constituant | Formule unitaire |
|---|---|
| Fumagilline | 20 mg |
| Aerosil® R972 | 25 mg |
| Labrafac®PG | 475 mg |

pour une gélule Licaps orange suédois op.C314 taille 0 remplie à 520 mg

### EXEMPLE 6 :

| Constituant | Formule unitaire |
|---|---|
| Fumagilline | 20 mg |
| Labrafac®PG | QSP 5 ml |

Ampoule buvable verre brun de 5 ml

### EXEMPLE 7 :

| Constituant | Formule unitaire |
|---|---|
| Fumagilline | 200 mg |
| Labrafac®PG | QSP 10 ml |

Flacon multidoses verre brun de 10 ml

### EXEMPLE 8 :

| Constituant | Formule unitaire |
|---|---|
| Fumagilline | 20 mg |
| Aerosil® R972 | 25 mg |
| Labrafac®PG | 475 mg |

Pour une capsule molle blanc-opaque terminée à 520 mg

### EXEMPLE 9 :

| Constituant | Formule unitaire |
|---|---|
| Fumagilline | 20 mg |
| Aerosil® R972 | 50 mg |
| Labrafac®PG | 950 mg |

Gel rectal en récipient unidose avec canule

### EXEMPLE 10 :

| Constituant | Formule unitaire |
|---|---|
| Fumagilline | 20 mg |
| Aerosil® R972 | 250 mg |
| Labrafac®PG | 4750 mg |

Gel rectal en récipient unidose avec canule

### EXEMPLE 11 :

| Constituant | Formule unitaire |
|---|---|
| Fumagilline | 20 mg |
| Labrafac®PG | 215 mg |

Pour une gélule Licaps "blanc-opaque" taille 1 remplie à 235 mg

### EXEMPLE 12 :

| Constituant | Formule unitaire |
|---|---|
| Fumagilline | 20 mg |
| Aerosil® R972 | 16 mg |
| Labrafac®PG | 214 mg |

Pour une gélule Licaps "blanc-opaque" de taille 1 remplie à 250 mg.

### EXEMPLE 13

| Constituant | Formule unitaire |
|---|---|
| Fumagilline | 20 mg |
| Labrafac®PG | 215 mg |

Pour une gélule Licaps "blanc-opaque" taille 1 remplie à 235 mg contenue dans une gélule Licaps "blanc-opaque" taille 0

### EXEMPLE 14 :

Gélule Licaps "blanc-opaque" taille 1 selon l'EXEMPLE 11 ou 12 banderolée avec la formule de banderolage suivante :

**Formule de banderolage pour une gélule**

| Constituant | Formule unitaire |
|---|---|
| Gélatine 200 blooms | 0,192 mg |
| Eau purifiée * | 2,208 mg |

| | |
|---|---|
| * éliminée au cours du procédé (séchage) | |

### EXEMPLE 15 :

Gélule Licaps "blanc-opaque" de taille 1 selon l'EXEMPLE 11 ou 12 scellée avec la formule de banderolage suivante :
formule de scellage (machine LEMS) : solution hydro-alcoolique (50/50) (éliminée au cours du procédé).

Une solution hydro-alccolique est pulvérisée sur les gélules et un séchage est effectué à une température de 44°C. L'eau abaisse la Tg (transition vitreuse) de la gélatine. L'alcool abaisse la tension superficielle et permet l'ascension capillaire entre le corps et la coiffe. L'augmentation de température permet l'évaporation et la fusion de la gélatine sur elle-même.

## Revendications

1. Formulation stable contenant à titre de principe actif de la fumagilline, **caractérisée en ce que** la fumagilline est stabilisée par un agent hydrophobe consistant en propylèneglycol dicaprate/dicaprylate.

2. Formulation selon la revendication 1 **caractérisée en ce qu'**elle contient un agent thixotrope.

3. Formulation selon la revendication 2 **caractérisée en ce que** l'agent thioxotrope est la silice colloïdale.

4. Formulation selon l'une des revendications 1 à 3 **caractérisée en ce qu'**elle est administrable par voie orale.

5. Formulation selon la revendication 4 **caractérisée en ce qu'**elle se présente sous la forme d'une gélule ou d'une capsule.

6. Formulation selon la revendication 5 **caractérisée en ce qu'**elle se présente sous la forme d'une capsule ou d'une gélule scellée.

7. Formulation selon la revendication 5 **caractérisée en ce qu'**elle se présente sous la forme d'une capsule ou d'une gélule banderollée.

8. Formulation selon l'une quelconque des revendications 5 à 7 dans laquelle la gélule ou la capsule est non-translucide.

9. Formulation selon la revendication 4 **caractérisée en ce qu'**elle est buvable.

10. Formulation selon la revendication 9 **caractérisée en ce qu'**elle se présente sous la forme d'un sirop conservé en flacon multidose ou en ampoule unidose non-translucide.

11. Formulation stable selon l'une des revendications 1 à 3, **caractérisée en ce qu'**elle est administrable par voie rectale.

12. Formulation selon l'une quelconque des revendications 1 à 11 **caractérisée en ce qu'**elle contient 0,2 à 15%(p/p) de fumagilline.

13. Formulation selon l'une des revendications 1 à 8 **caractérisée en ce qu'**elle contient 8 à 10% (p/p) de fumagilline.

14. Formulation pharmaceutique selon la revendication 13 **caractérisée en ce qu'**elle contient 20 mg de fumagilline et 215 mg de propylèneglycol dicaprate/dicaprylate dans une gélule.

15. Formulation pharmaceutique selon l'une quelconque des revendications 1 à 11 **caractérisée en ce qu'**elle contient 5 à 12% (p/p) de fumagilline.

16. Formulation pharmaceutique selon l'une quelconque des revendications 1 à 8 **caractérisée en ce qu'**elle contient 20 mg de fumagilline et 400 mg de propylèneglycol dicaprate/dicaprylate dans une gélule.

17. Formulation pharmaceutique selon l'une quelconque des revendications 1 à 8 **caractérisée en ce qu'**elle contient 20 mg de fumagilline, 25 mg de silice colloïdale et 475 mg de propylèneglycol dicaprate/dicaprylate dans une capsule ou une gélule.

18. Formulation pharmaceutique selon la revendication 17 **caractérisée en ce que** la quantité de silice colloïdale est de 11.75 mg et celle de propylèneglycol dicaprate/dicaprylate de 223,25 mg dans une gélule.

19. Formulation pharmaceutique selon la revendication 17 **caractérisée en ce que** la quantité de silice colloïdale est de 16 mg et celle de propylèneglycol dicaprate/dicaprylate est de 214 mg dans une gélule.

20. Formulation pharmaceutique selon la revendication 9 ou 10 **caractérisée en ce qu'**elle contient 20 mg ou 200 mg de fumagilline.

21. Formulation pharmaceutique selon la revendication 11 **caractérisée en ce qu'**elle contient 20 mg de fumagilline 50 mg de silice colloïdale et 950 mg de propylèneglycol dicaprate/dicaprylate.

22. Formulation pharmaceutique selon la revendication 11 **caractérisée en ce qu**'elle contient 20 mg de fumagilline et en ce que la quantité de silice colloïdale est de 250 mg et la quantité de propylèneglycol dicaprate/dicaprylate est de 4750 mg.

23. Utilisation de la formulation selon l'une quelconque des revendications 1 à 14 pour la fabrication de médicaments destinés à traiter et/ou combattre les infections intestinales dues à des microsporidies et/ou à des cryptosporidies.

## Claims

1. Stable formulation, containing fumagillin as active principle, **characterized in that** the fumagillin is stabilized by a hydrophobic agent consisting of propylene glycol dicaprate/dicaprylate.

2. Formulation according to Claim 1, **characterized in** chat it contains a thixotropic agent.

3. Formulation according to Claim 2, **characterized in that** the thixotropic agent is colloidal silica.

4. Formulation according to one of Claims 1 to 3, **characterized in that** it is orally administrable.

5. Formulation according to Claim 4, **characterized in that** it is in the form of a gel capsule or a capsule.

6. Formulation according to Claim 5, **characterized in that** it is in the form of a sealed capsule or gel capsule.

7. Formulation according to Claim 5, **characterized in that** it is in the form of a hoop cased capsule or gel capsule.

8. Formulation according to any one of Claims 5 to 7, in which the gel capsule or the capsule is nontranslucent.

9. Formulation according to Claim 4, **characterized in that** it is drinkable.

10. Formulation according to Claim 9, **characterized in that** it is in the form of a syrup stored in a nontranslucent multidose bottle or single dose ampule.

11. Stable formulation according to one of Claims 1 to 3, **characterized in that** it is rectally administrable.

12. Formulation according to any one of Claims 1 to 11, **characterized in that** it contains 0.2% to 15% (w/w) of fumagillin.

13. Formulation according to one of Claims 1 to 8, **characterized in that** it contains 8% to 10% (w/w) of fumagillin.

14. Pharmaceutical formulation according to Claim 13, **characterized in that** it contains 20 mg of fumagillin and 215 mg of propylene glycol dicaprate/dicaprylate in a gel capsule.

15. Pharmaceutical formulation according to any one of Claims 1 to 11, **characterized in that** it contains 5% to 12% (w/w) of fumagillin.

16. Pharmaceutical formulation according to any one of Claims 1 to 8, **characterized in that** it contains 20 mg of fumagillin and 400 mg of propylene glycol dicaprate/dicaprylate in a gel capsule.

17. pharmaceutical formulation according to any one of Claims 1 to 8, **characterized in that** it contains 20 mg of fumagillin, 25 mg of colloidal silica and 475 mg of propylene glycol dicaprate/dicaprylate in a capsule or gel capsule.

18. Pharmaceutical formulation according to Claim 17, **characterized in that** the amount of colloidal silica is 11.75 mg and that of propylene glycol dicaprate/dicaprylate is 223.25 mg in a gel capsule.

19. Pharmaceutical formulation according to Claim 17, **characterized in that** the amount of colloidal silica is 16 mg and that of propylene glycol dicaprate/dicaprylate is 214 mg in a gel capsule.

20. Pharmaceutical formulation according to Claim 9 or 10, **characterized in that** it contains 20 mg or 200 mg of fumagillin.

21. Pharmaceutical formulation according to Claim 12, **characterized in that** it contains 20 mg of fumagillin. 50 mg of colloidal silica and 950 mg of propylene glycol dicaprate/dicaprylate.

22. Pharmaceutical formulation according to Claim 11, **characterized in that** it contains 20 mg of fumagillin and that the amount of colloidal silica is 250 mg and that of propylene glycol dicaprate/dicaprylate is 4750 mg.

23. Use of the formulation according to any one of Claims 1 to 14 to manufacture medicinal products for treating and/or combating intestinal infections caused by microsporidae and/or cryptosporidae.

## Patentansprüche

1. Stabile Formulierung, die als Wirkstoff Fumagillin enthält, **dadurch gekennzeichnet, dass** das Fumagillin durch ein hydrophobes Mittel stabilisiert wird, das aus Propylenglycoldicaprat/dicaprylat besteht.

2. Formulierung nach Anspruch 1, **dadurch gekennzeichnet, dass** sie ein thixotropes Mittel enthält.

3. Formulierung nach Anspruch 2, **dadurch gekennzeichnet, dass** das thixotrope Mittel kolloidales Siliciumdioxid ist.

4. Formulierung nach einem der Ansprüche 1 bis 3, **dadurch gekennzeichnet, dass** sie oral verabreichbar ist.

5. Formulierung nach Anspruch 4, **dadurch gekennzeichnet, dass** sie in Form einer Gelatinekapsel oder einer Kapsel dargereicht wird.

6. Formulierung nach Anspruch 5, **dadurch gekennzeichnet, dass** sie in Form einer Kapsel oder einer versiegelten Gelatinekapsel dargereicht wird.

7. Formulierung nach Anspruch 5, **dadurch gekennzeichnet, dass** sie in Form einer Kapsel oder einer banderolierten Gelatinekapsel dargereicht wird.

8. Formulierung nach einem der Ansprüche 5 bis 7, wobei die Gelatinekapsel oder die Kapsel nicht lichtdurchlässig ist.

9. Formulierung nach Anspruch 4, **dadurch gekennzeichnet, dass** sie trinkbar ist.

10. Formulierung nach Anspruch 9, **dadurch gekennzeichnet, dass** sie dass sie in Form eines Sirups dargereicht wird, der in Mehrfachdosis-Glasfläschchen oder in einer lichtundurchlässigen Einzeldosis-Ampulle aufbewahrt wird.

11. Formulierung nach einem der Ansprüche 1 bis 3, **dadurch gekennzeichnet, dass** sie rektal verabreichbar ist.

12. Formulierung nach einem der Ansprüche 1 bis 11, **dadurch gekennzeichnet, dass** sie 0,2 bis 15 % (Gew./Gew.) Fumagillin enthält.

13. Formulierung nach einem der Ansprüche 1 bis 8, **dadurch gekennzeichnet, dass** sie 8 bis 10 % (Gew./Gew.) Fumagillin enthält.

14. Pharmazeutische Formulierung nach Anspruch 13, **dadurch gekennzeichnet, dass** sie 20 mg Fumagillin und 215 mg Propylenglycoldicaprat/dicaprylat in einer Gelatinekapsel enthält.

15. Pharmazeutische Formulierung nach einem der Ansprüche 1 bis 11, **dadurch gekennzeichnet, dass** sie 5 bis 12 % (Gew./Gew.) Fumagillin enthält.

16. Pharmazeutische Formulierung nach einem der Ansprüche 1 bis 8, **dadurch gekennzeichnet, dass** sie 20 mg Fumagillin und 400 mg Propylenglycoldicaprat/dicaprylat in einer Gelatinekapsel enthält.

17. Pharmazeutische Formulierung nach einem der Ansprüche 1 bis 8, **dadurch gekennzeichnet, dass** sie 20 mg Fumagillin, 25 mg kolloidales Siliciumdioxid und 475 mg Propylenglycoldicaprat/dicaprylat in einer Kapsel oder einer Gelatinekapsel enthält.

18. Pharmazeutische Formulierung nach Anspruch 17, **dadurch gekennzeichnet, dass** die Menge an kolloidalem Siliciumdioxid 11,75 mg und diejenige an Propylenglycoldicaprat/dicaprylat 223,25 mg in einer Gelatinekapsel beträgt.

19. Pharmazeutische Formulierung nach Anspruch 17, **dadurch gekennzeichnet, dass** die Menge an kolloidalem Siliciumdioxid 16 mg und diejenige an Propylenglycoldicaprat/dicaprylat 214 mg in einer Gelatinekapsel beträgt.

20. Pharmazeutische Formulierung nach Anspruch 9 oder 10, **dadurch gekennzeichnet, dass** sie 20 mg oder 200 mg Fumagillin enthält.

21. Pharmazeutische Formulierung nach Anspruch 11, **dadurch gekennzeichnet, dass** sie 20 mg Fumagillin, 50 mg kolloidales Siliciumdioxid und 950 mg Propylenglycoldicaprat/dicaprylat enthält.

22. Pharmazeutische Formulierung nach Anspruch 11, **dadurch gekennzeichnet, dass** sie 20 mg Fumagillin enthält und dass die Menge an kolloidalem Siliciumdioxid 250 mg und die Menge an Propylenglycoldicaprat/dicaprylat 4750 mg beträgt.

23. Verwendung der Formulierung nach einem der Ansprüche 1 bis 14 zur Herstellung von Medikamenten, die zur Behandlung und/oder Bekämpfung von Darminfektionen auf Grund von Mikrosporidien und/oder Kryptosporidien bestimmt sind.
